# EUROPÄISCHE PATENTSCHRIFT

(11) **EP 0 888 757 B1**
(45) Veröffentlichungstag und Bekanntmachung des Hinweises auf die Patenterteilung: **21.01.2004**
(21) Anmeldenummer: 97810417.2
(22) Anmeldetag: 30.06.1997
(51) Int. Cl.: A61F 2/06

(54) **Intraluminales Implantat**
Intraluminal implant
Implant intraluminaire

(43) Veröffentlichungstag der Anmeldung: 07.01.1999
(73) Patentinhaber: Medex Holding GmbH, 40549 Düsseldorf (DE)
(72) Erfinder: Hassdenteufel, Hans, 55128 Mainz (DE)
(74) Vertreter: Behrens, Dieter, Dr.-Ing.

(56) Entgegenhaltungen:
- WO-A-96/03092
- WO-A-96/26689
- DE-A- 4 418 336
- DE-U- 29 702 671
- US-A- 5 195 984

## Beschreibung

Die Erfindung betrifft ein intraluminales Implantat (Stent) zur Aufweitung eines Blutgefässes gemäss Oberbegriff des Patentanspruches 1.

Intraluminale Implantate zur Aufweitung von Blutgefässen, die einen ersten Durchmesser aufweisen und nach Plazierung im Blutgefäss auf einen zweiten Durchmesser aufgeweitet werden können, sind seit 1978 bekannt. Bei den ältesten Implantaten handelt es sich um einfache, wendelförmig gewickelte Drahtspulen, die radial nach innen vorgespannt eingebracht werden und an der gewünschten Stelle im Lumen losgelassen werden, so dass sie sich radial nach außen entspannen können und dabei die Gefässwand radial nach aussen drücken. Die Applikation federelastischer Stents wurde mit der Verbreitung der Ballonkatheter ersetzt durch plastisch verformbare Implantate.

Diese Implantate sind im wesentlichen in drei Grundtypen vorhanden. Gewickelte endlose Drahtgebilde sind aus den Dokumenten EP-A-0'282'175, EP-A-0'312'852, EP-A-0'378'151 oder US-A-5'133'732, US-A-5'135'536 und US-A-5'562'697 oder DE-A-44'32'938, um nur einige zu nennen, bekannt. Diese Stents sind nicht sehr formstabil und sind entsprechend diffizil in Bezug auf das Aufbringen auf den Ballonkatheter.

Die weitaus grösste Anzahl intraluminaler Implantate besteht aus einem Geflecht aus mehreren Drähten, die äusserst aufwendig geformt und stellenweise durch Punktschweissung versteift sind. Eine Auswahl solcher Stents zeigen die EP-A-0'421'729, EP-A-0'423'916, EP-A-0'480'667, EP-A-565'251 oder EP-A-0'744'164, um nur einige zu nennen. Herstellungstechnisch sind diese Implantate äusserst aufwendig und entsprechend teuer.

Der hier interessierende plastish verformbare Stent ist ein intraluminales Implantat, welches aus einem dünnwandigen Rohr mittels einem Laser zu einem maschigen Gebilde ausgeschnitten wird. Solche Implantate haben den grossen Vorteil, dass sie eine absolut glatte zylindrische Oberfläche haben im nicht aufgeweiteten Zustand und sich folglich leicht auf einen Ballonkatheter aufbringen lassen und auch keinen relevanten Reibungswiderstand bei der Einführung und dem Transport in einem Gefäss bewirken.

Die verbleibenden flachen Stege haben eine Dicke, die wesentlich unter der Dicke von Drähten für geflochtene Stents mit vergleichbarem Durchmesser liegt. Entsprechend reduziert sich der Gesamtdurchmesser des Implantates, was den Einsatz in Gefässen mit geringerem Innendurchmesser erlaubt.

Eine erste Ausführung gemäss der EP-B-0'221'570 zeigt ein Implantat, das aus einem Rohrstück besteht, in dem lauter in achsialer Richtung verlaufende rechteckige Ausnehmungen ausgelasert sind. Im nicht aufgeweiteten Zustand ist ein solches Implantat praktisch biegesteif. Dies begrenzt die Länge des Stents und verlangt gegebenenfalls die Setzung von mehreren aufeinanderfolgenden Stents. Diese nicht überlappend, jedoch relativ dicht aufschliessend anzubringen, ist heikel. Entsprechend sieht eine weiterentwickelte Lösung vor, dass der an sich gleich gestaltete Stent aus mehreren über Steg je einen geraden einstückig miteinander verbundenen Abschnitten besteht, wie dies aus der US-A-5'195'984 hervorgeht, die den Oberbegriff des Patentanspruches 1 bildet. Die Flexibilität dieser Lösung entspricht einer groben Gliederkette und neigt zur Knickung.

Bei allen bekannten, aus einem Rohr gefertigten intraluminalen Implantaten erfolgt bei der Aufweitung des Durchmessers eine Verkurzung in achsialer Richtung. Diese Verkürzung ist schwer abzuschätzen, obwohl sie in direktem Bezug zur Aufweitung steht. Dies erschwert dem Arzt die exakte Wahl des richtigen Stents und dessen korrekte Plazierung.

Dies gilt auch für einen bekannten Stent (WO 96/26689), der mehrere Ringe aus mäanderförmig verlaufenden Materialbahnen hat. Die äußeren Ringe von z. B. sechs benachbarten Ringen können breiter als die inneren Ringe sein. Die Ringe selbst sind jeweils durch drei gegenüber der Mittelachse der Ringe schräg verlaufende gerade Stege miteinander verbunden, die jeweils angular um 120° gegeneinander versetzt sind. Bei einem anderen, ähnlich aufgebauten Stent (DE 44 18 336 A1) sind kurze achsparallele Verbindungsstege zwischen benachbarten Ringen vorgesehen. Schließlich ist ein Stent bekannt (DE 297 02 671), bei dem die Ringe aus einer Vielzahl von aneinandergrenzenden, haarnadelartig ausgebildeten Elementen bestehen, die eine angenähert mäanderförmig verlaufende Materialbahn bilden. Die Ringe sind durch viele einen Scheitelpunkt und einen Fußpunkt aufweisende Stegbögen miteinander verbunden. Bei allen diesen bekannten Stents ist eine nur geringe axiale Beweglichkeit zu beobachten. Beim Aufweiten des Stents tritt eine deutlich Verkürzung der Gesamtlänge auf, da die Verbindungsstege zwischen den Stegen dabei nicht oder praktisch nicht gestreckt werden. Die dazu erforderliche Axial- bzw. Reibkraft kann beim Aufweiten nicht vom Ballonkatheter erzeugt werden.

Der Erfindung liegt die Aufgabe zugrunde, ein luminales Implantat (Stent) der zuletzt genannten Art, das nach dem Einführen in ein Gefäß radial plastisch aufgeweitet wird, derart zu verbessern, daß es im nicht-aufgeweiteten Zustand in axialer Richtung flexibel ist, dabei aber bei der Aufweitung keine relevante Längenänderung erfährt. Nach einer Aufweitung soll es ausreichende elastische Eigenschaften besitzen, um den Verformungen der Koronargefäße bei einem Herzschlag zu entsprechen.

Diese Aufgabe löst ein Implantat mit den Merkmalen des Patentanspruchs 1. Vorteilhafte Ausgestaltungen ergeben sich aus den Unteransprüchen.

In der Zeichnung ist eine bevorzugte Ausführungsform der Erfindung stark vereinfacht dargestellt. Es zeigt:
- Fig. 1: einen Abschnitt eines luminalen Implantates in der Seitenansicht im nicht aufgeweiteten Zustand, in dem es in ein Gefäß eingeführt wird, und
- Fig. 2: denselben Abschnitt des Implantates im aufgeweiteten Zustand.
- Figur 3: zeigt einen Vertikalschnitt senkrecht zur Längsachsrichtung entlang der Linie III in Figur 2 und
- Figur 4: einen ebensolchen Schnitt entlang der Linie IV wie in Figur 2 dargestellt;
- Figur 5: stellt eine Abwicklung einer weiteren Ausgestaltungsform dar, ebenso wie
- Figur 6: eine Abwicklung eines kürzeren Stentes zeigt.

Das erfindungsgemässe intraluminale Implantat wird vorzugsweise in Blutbahnen eingesetzt und hier wiederum bevorzugterweise in Koronargefässen. Wie bereits eingangs erwähnt, ist das hier interessierende intraluminale Implantat aus einem dünnwandigen, nahtlosen Rohr aus Stahl für chirurgische Implantate gefertigt. Aus diesem Rohr werden mit einem Laser Zwischenräume ausgeschnitten, so dass schlussendlich ein Gebilde ähnlich einem Netz beziehungsweise einem netzförmigen Strumpf entsteht. Diese verbleibenden Wandpartien haben somit die Dicke der ursprünglichen Wandstärke des Ausgangsrohres. Die hierzu verwendeten Wandstärken liegen üblicherweise zwischen 0,04 und 0,2 mm. Der Aussendurchmesser des nahtlosen Rohres liegt üblicherweise zwischen 1,0 und 5 mm. Die Breite der verbleibenden Wandbereiche oder Materialbahnen kann selbstverständlich ebenfalls variieren. In der Zeichnung ist das intraluminale Implantat, auch Stent genannt, vereinfacht in einer Seitenansicht dargestellt. Alle verbliebenen Materialbahnen sind nur als einfache Linien dargestellt. In Realität haben sie selbstverständlich eine flächige Ausdehnung, wie dies oben erklärt ist. Der Einfachheit halber wird auf diese flächige Darstellung jedoch verzichtet. Wegen der Durchbrüche, die durch das Weglasern entstehen, wären prinzipiell die Materialbereiche auf der von der Ansichtsseite her entfernt gelegenen Seite des rohrförmigen Gebildes auch sichtbar. Auf deren Darstellung wurde jedoch verzichtet. Somit stellt eigentlich die Seitenansicht nur eine Ansicht eines Halbrohres dar.

Die Länge eines Stents kann prinzipiell variieren. Einerseits wird er entsprechend der medizinischen Indikation dimensioniert und andererseits wird seine minimale Grösse und maximale Länge durch den verwendeten Ballonkatheter begrenzt. In der Zeichnung ist lediglich ein Abschnitt des intraluminalen implantates dargestellt. Das gesamte intraluminale Implantat 1 besteht aus einer Vielzahl expandierbarer Ringe 2, die lauter achsiale Abschnitte des insgesamt zylindrischen Stents darstellen. Diese Ringe oder Ringabschnitte 2 sind expandierbar. Die Aufweitung in radialer Richtung erfolgt wie üblich mittels eines Ballonkatheters. Jeder expandierbare Ring 2 kann aus einem oder aus mehreren Reifen 3 bestehen, deren Materialbahnen in der Fläche mäanderförmig verlaufenden. Unter mäanderformig verlaufenden Materialbahnen werden hier solche verstanden, die in der zylindrischen Fläche, die sie aufspannen, zickzack, sinusförmig oder anders gewellt verlaufen können. Im hier dargestellten Beispiel verlaufen diese Materialstreifen wellen -oder mäander förmig. Hierbei sind die einander entgegengerichteten Kuppen benachbarter Reifen 3 desselben Ringes 2 über Stege 5 miteinander verbunden. Diese Stege verlaufen in achsialer Richtung in der zylindrischen Mantelfläche gekrümmt, beispielsweise zu einem Spitzbogen. Dank diesen Verbindungen sind die einzelnen Reifen 3 relativ zueinander elastisch und plastisch verformbar gehalten, bewirken jedoch trotzdem eine genügende Festigkeit, damit das ohnehin äusserst filigrane Gebilde nicht schon beim Aufsetzen auf den Dilatator zerstört wird. Jeweils zwei benachbarte Ringe 2 sind über Stege 4 ebenfalls miteinander verbunden. Um eine grösstmögliche Flexibilität des Stents zu erreichen, wird diese Verbindung auf ein absolutes Minimum reduziert, um die achsiale Flexibilität des Stents zu optimieren. Zwischen zwei benachbarten Ringen sind zwei Stege 4 ausgebildet, wobei diese jedoch nicht einander diametral gegenüber angeordnet sind sondern einen stumpfen Winkel, vorzugsweise von etwa 120° zwischen einander einschliessen. Die Stege 4 zwischen aufeinanderfolgenden Ringen 2 werden jeweils um einen bestimmten Winkel versetzt angeordnet. Durch diese Anordnung ist die Biegsamkeit des Stents in jeder von der achsialen Richtung abweichenden Richtung etwa gleich gross.

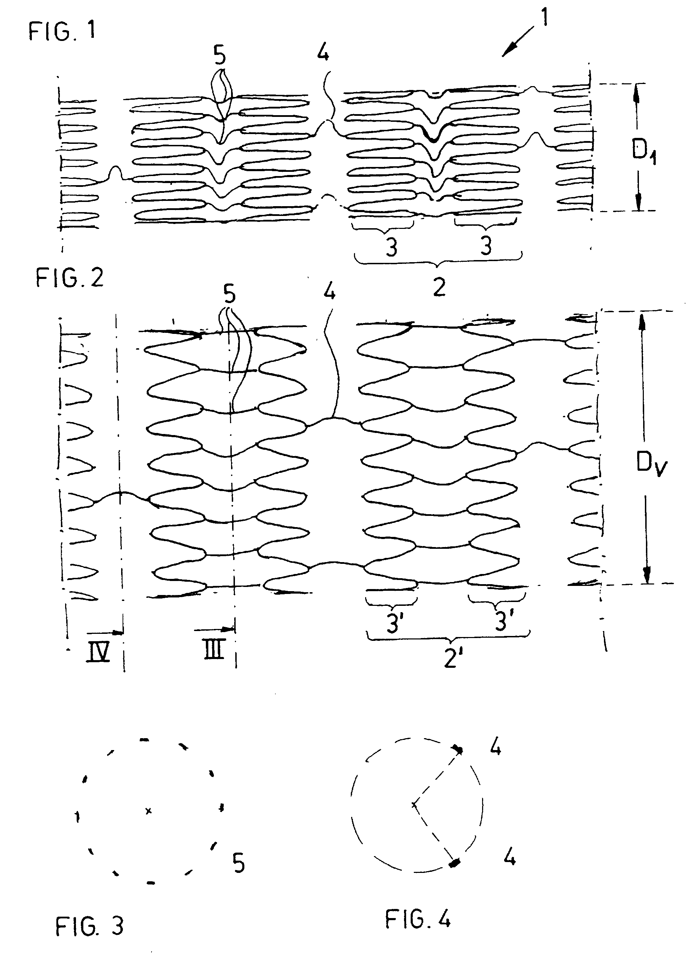

Während der Abschnitt des intraluminales Implantates gemäss der Figur 1 einen ersten Durchmesser von D₁ aufweist, hat derselbe Abschnitt in Figur 2 den aufgeweiteten Durchmesser Dᵥ. In der Zeichnung erkennt man, dass bei der Aufweitung die Erstreckung in achsialer Richtung sich praktisch kaum verändert hat. Zwar reduziert sich bei der Aufweitung des Durchmessers von D₁ auf Dᵥ die Breite der Reifen 3 auf die Breite der Reifen 3', doch ist diese Breitenveränderung relativ minimal und wird durch die Streckung der streckbaren Stege 5 zwischen den Reifen weitgehend kompensiert. Die leichte Verkürzung der Reifen 3 führt zu einer ebenfalls geringfügigen Verkürzung der Ringe 2 in achsialer Richtung zu einer Länge 2'. Diese Längenänderung wird durch die zwei streckbaren Stege 4 leicht aufgefangen. Auch die Stege 4 haben eine spitzbogenförmige Gestaltung. Es hat sich dabei gezeigt, dass es von Vorteil ist, die Spitzbogenrichtung der Stege 4 zwischen benachbarten Ringen und die Spitzbogen der Stege 5 zwischen benachbarten Reifen 3 einander entgegengesetzt auszurichten.

Wegen der hier gewählten Darstellungsform geht die Dreidimensionalität des Implantates 1 verloren. Aus den Figuren 3 und 4, welche Vertikalschnitte durch den Stent entlang den Linien III beziehungsweise IV in der Figur 2 zeigen, ersieht man die zylindrische Gestaltung. Beim Schnitt entlang der Linie III gemäss der Figur 3 werden sämtliche Stege 5 zwischen zwei benachbarten Reifen 3 desselben Ringes 2 geschnitten. Leicht übertrieben ist die etwa flächige Ausdehnung der Stege erkennbar. Beim Schnitt gemäss der Figur 4 werden die lediglich zwei streckbaren Stege 4 zwischen zwei benachbarten Ringen 2 gezeigt. Die beiden Stege 4 schliessen einen Winkel α ein. Dieser Winkel wird vorzugsweise zwischen 90 und 180° gewählt. Im darauffolgenden Spaltbereich zwischen zwei benachbarten Ringen 2 sind diese beiden Stege 4 unter Beibehaltung des Winkels α um einen gewissen Winkel versetzt angeordnet. In einer gewählten Ausführungsform wurde der Winkel α mit 120° bestimmt und der Versetzungswinkel der Stege aufeinanderfolgender Ringe wurde mit 60° festgelegt. Die Wahl dieser Winkel kann jedoch variiert werden.

Implantate stellen immer einen Fremdkörper im Patienten dar. Entsprechend wünscht man diesen so klein wie möglich und so gross wie nötig zu gestalten. Da die Gesamtlänge des Implantates gemäss der Erfindung sich praktisch nicht verändert, braucht der Arzt keinen Verkürzungsfaktor des Implantates mitzuberücksichtigen, wenn er das erfindungsgemässe intraluminale Implantat verwendet. Bei der Aufweitung des Stents dehnt sich der Ballon des Ballonkatheters dort am stärksten aus, wo er am wenigsten Widerstände findet. Im vorliegenden Fall ist dies immer der Bereich zwischen zwei benachbarten Ringen und im verminderten Auflagebereich am Umfang zwischen zwei benachbarten Reifen. Durch diese Tatsache werden beim Aufblasen des Ballones die Ringe beziehungsweise die Reifen mehr oder weniger stark in achsialer Richtung positioniert. Entsprechend werden die achsial auftretenden Kräfte zu einer Streckung der Stege 4 beziehungsweise 5 führen und nicht lediglich eine Verschiebung der Ringe 2 beziehungsweise der Reifen 3 im Lumen oder auf dem Ballon des Katheters zur Folge haben.

Die Aufweitung des Implantates ohne wesentliche Längenveränderung stellt sicher, dass bei der Aufweitung des Stentes nicht Verschiebungen der Ringe auftreten, die Verletzungen der Gefässwand bewirken können.

Die ausgesprochen starke Flexibilität des intraluminalen Implantates in jeder von der achsialen Richtung abweichenden Richtung ist derart groß, dass sich nach der Entfernung des Ballonkatheters der Stent sogar noch nachträglich dem Verlauf des Lumens anpassen kann, so dass gefürchtete Knickstellen vollkommen vermieden werden. Insbesondere wenn benachbarte Ringe 2 mit zwei Verbindungsstegen 4 miteinander verbunden sind, ist die Biegsamkeit des Stentes besser, wenn die beiden Stege nicht diagonal einander gegenüberliegend angeordnet sind, sondern einen kleineren Winkel von beispielsweise 120° einschliessen.

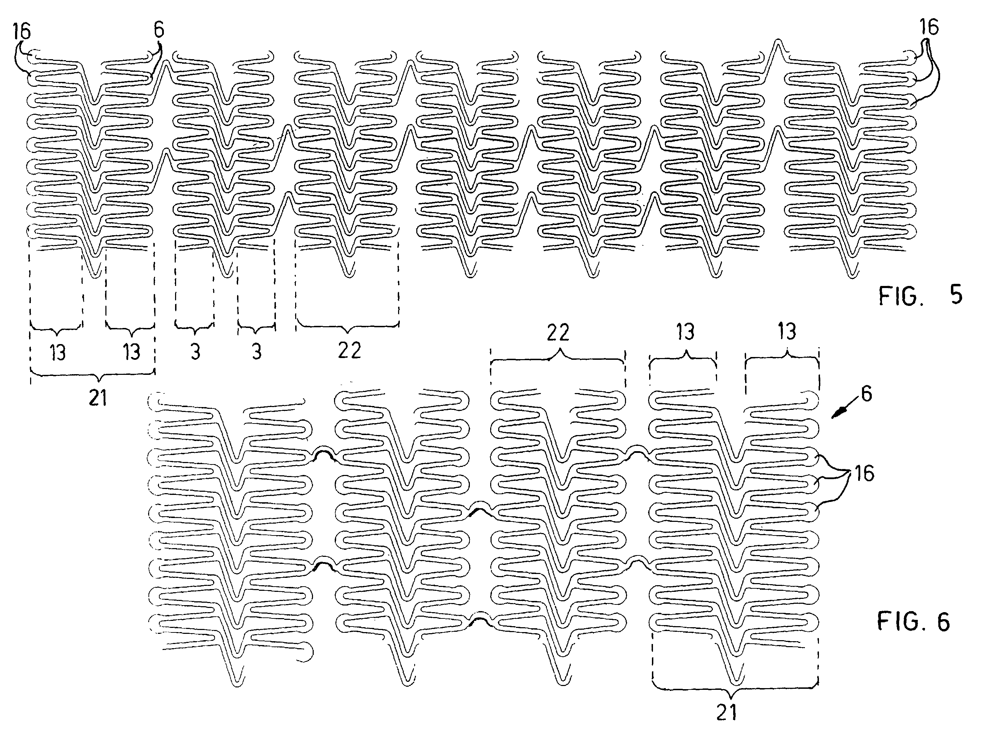

Zwei weitere Ausgestaltungsformen sind in den Figuren 5 und 6 dargestellt. Die hier dargestellten Stents sind Abwicklungen der dünnwandigen, mittels Laser geschnittenen intraluminalen Implantate. Das Besondere dieser Ausführung besteht darin, dass die Breite der beiden zuäusserst liegenden Reifen 13 grösser ist als die Breite der übrigen Reifen. Entsprechend ist auch die Breite der beiden zuäusserst liegenden Ringe 21 grösser als die Breite der dazwischen angeordneten Ringe 22. Die Breite der Reifen bzw. die Breite der Ringe steht im umgekehrten Verhältnis zur erforderlichen Kraft, um diese in radialer Richtung auf die gewünschte Grösse aufzuweiten. Durch diese Gestaltung soll sichergestellt werden, dass das expandierte Implantat auch an den äussersten Ringbereichen satt an der Lumenwandung anliegt. Der Ballon eines Ballonkatheters neigt prinzipiell eher dazu, mittig stärker auszubauchen als an den Randbereichen. Die hier vorgeschlagene Ausgestaltungsform des Implantates trägt diesem Effekt Rechnung, wobei die Breite der beiden jeweils äussersten Reifen 13 sogar so breit gewählt wird, dass eine gewisse Ueberkompensation vorhanden ist, so dass der Stent insbesondere in den Randbereichen eher stärker ausgeweitet wird als in der Mitte.

Bei der Aufweitung der mäandernden Reifen bzw. Ringe werden die Kuppen 6 der Reifen 13 etwas stärker radial nach aussen gedrückt als die restlichen Bereiche, wobei verformungsbedingt eine gewisse Torsion entsteht, die einem flächigen Aufliegen der Kuppen auf der Lumenwandung entgegenwirkt. Um einen solchen Effekt insbesondere bei den zuäusserst liegenden Kuppen 6 zu vermeiden, sind hier die Kuppen mit flachigen Verbreiterungen 16 versehen. Insbesondere für Stents, die für Lumen mit größeren Durchmessern verwendet werden, kann es dann auch sinnvoll sein, sämtliche Kuppen 6, mit Ausnahme jener Kuppen, die über Stege 4,5 miteinander verbunden sind, flächig zu verbreitern. Diese Lösung ist in der Figur 6 dargestellt. Aus dem eben Erwähnten ergibt sich, dass der Stent gemäss der in Figur 5 dargestellten Abwicklung ein zwar in achsialer Richtung sich länger ausdehnender Stent ist als in Figur 6, dass jedoch der Stent nach Figur 5 für ein Lumen mit geringerem Innendurchmesser geeigneter ist als der Stent nach Figur 6.

## Patentansprüche

1. Intraluminales Implantat (1) zur Aufweitung eines Blutgefäßes aus einem dünnen rohrförmigen Teil,
das aus einem nahtlosen dünnwandigen Metallrohr so geschnitten ist, daß expandierbare Ringe (2, 21, 22) aus netzartig oder mäanderförmig verlaufenden verbleibenden Materialbahnen entstanden sind, die miteinander durch Verbindungsstege (4) verbunden sind, wobei in axialer Richtung folgende Verbindungsstege (4) gegenüber den vorhergehenden angular zueinander versetzt angeordnet sind;
das einen ersten Durchmesser (D₁) aufweist, der dem intraluminalen Transport des rohrförmigen Teiles dient, das auf einen zweiten, variablen Durchmesser (Dv) dadurch aufgeweitet werden kann, daß vom Inneren des rohrförmigen Teiles aus eine radiale, nach außen gerichtete Kraft aufgebracht wird; und
das mittels eines Ballonkatheters in das Blutgefäß transportiert und dort aufgeweitet wird,
**dadurch gekennzeichnet,**
**daß** jeweils zwei benachbarte Ringe (2, 21, 22) über genou zwei, in Axialrichtung des rohrförmigen Teils gekrümmt verlaufende streckbare Verbindungsstege (4) miteinander verbunden sind, die um weniger als 180° angular zueinander versetzt angeordnet sind.

2. Intraluminales Implantat nach Anspruch 1,
**dadurch gekennzeichnet, daß** die zwei jeweils zwei benachbarte Ringe miteinander verbindenden Verbindungsstege (4) um 60° bis 120° angular zueinander versetzt sind.

3. Intraluminales Implantat nach Anspruch 1 oder 2,
**dadurch gekennzeichnet, daß** die Verbindungsstege (4) spitzbogenförmig gekrümmt sind.

4. Intraluminales Implantat nach einem der Ansprüche 1 - 3,
**dadurch gekennzeichnet, daß** jeder expandierbare Ring (2) aus mindestens zwei Reifen (3) aus mäanderförmig verlaufenden verbleibenden Materialbahnen besteht, die über eine Vielzahl von in axialer Richtung gekrümmt verlaufenden streckbaren weiteren Verbindungsstegen (5) miteinander verbunden sind.

5. Intraluminales Implantat nach Anspruch 4,
**dadurch gekennzeichnet, daß** die Verbindungsstege (4) zwischen benachbarten Ringen (2) und die weiteren Verbindungsstege (5) zwischen benachbarten Reifen (3) beide spitzbogenförmig gekrümmt sind und die Spitzbögen zwischen zwei benachbarten Ringen (2) in die eine erste Richtung und die Spitzbögen zwischen zwei benachbarten Reifen (3) in die entgegengesetzte Richtung weisen.

6. Intraluminales Implantat nach einem der Ansprüche 1 - 5,
**dadurch gekennzeichnet, daß** mindestens die in axialer Richtung zuäußerst liegenden Kuppen der mäanderförmig verlaufenden Materialbahnen der Ringe (2, 21) flächige Verbreiterungen (16) aufweisen.

7. Intraluminales Implantat nach Anspruch 1,
**dadurch gekennzeichnet, daß** die in axialer Richtung zuäußerst liegenden Ringe (21) eine größere axiale Länge aufweisen als die dazwischen liegenden Ringe (22).

8. Intraluminales Implantat nach Anspruch 4,
**dadurch gekennzeichnet, daß** mindestens die in axialer Richtung zuäußerst liegenden Reifen (13) eine größere axiale Länge aufweisen als die übrigen Reifen (3).

9. Intraluminales Implantat nach Anspruch 4,
**dadurch gekennzeichnet, daß** die die in axialer Richtung äußersten Ringe (21) bildenden Reifen (13) breiter gestaltet sind als die übrigen Reifen (3).

## Claims

1. Intraluminal implant (1) for expanding a blood vessel the implant consisting of a thin tubular member, the thin tubular member being cut from a seamless thin-walled metallic tube in such a manner that expandable rings (2, 2, 22) of net-like or meander-like webs result which are interconnected with each other by connecting webs (4), connecting webs (4) following each other in axial direction being angularly offset with respect to each other, the tubular member having a first diameter (D₁) for intraluminal conveyance of the tubular member, which may be expanded to a variable second diameter (Dᵥ) by exerting an outwardly directed force from inside the tubular member and which is inserted into the blood vessel by means of a balloon-tipped catheter and expanded therein,
**characterized in that**
any two adjacent rings (2, 21, 22) are connected with each other by exactly two connecting webs (4) which are stretchable in axial direction of the tubular member and which are disposed offset with respect to each other in angular direction by less than 180 degrees.

2. Intraluminal implant as claimed in claim 1,
**characterized in that** the two connecting webs (4) interconnecting adjacent rings (2, 21, 22) are disposed offset with respect to each other in angular direction by 60° to 120°.

3. Intraluminal implant as claimed in claim 1 or 2,
**characterized in that** the webs (4) have a curved configuration of ogival shape.

4. Intraluminal implant as claimed in any of claims 1 to 3, **characterized in that** each expandable ring (2) includes of at least two hoops (3) of net-like or meander-like webs, which hoops are connected to each other by a plurality of additional stretchable connecting webs (5) curved in axial direction.

5. Intraluminal implant as claimed in claim 4, **characterized in that** the connecting webs (4) between adjacent rings (2) and the additional connecting webs (5) between adjacent hoops (3) both are ogival, the ogives between two rings (2) being oriented in one direction and the ogives between two hoops (3) being oriented in the opposite direction.

6. Intraluminal implant as claimed in any of claims 1 to 5, **characterized in that** at least those cusps which are located axially outermost of the meander-like webs of the rings (2, 21) have wider flat portions (16).

7. Intraluminal implant as claimed in claim 1, **characterized in that** the outermost rings (21) in axial direction of the implant have a greater length than the rings (22) disposed in between.

8. Intraluminal implant as claimed in claim 4, **characterized in that** at least the axially outermost hoops (13) have a greater length than any other of the hoops (3).

9. Intraluminal implant as claimed in claim 4, **characterized in that** the hoops (13) forming the axially outermost rings (21) are longer than any other of the hoops (3).

## Revendications

1. Implant intraluminal (1) pour élargir un vaisseau sanguin, constitué par une partie tubulaire mince
qui est découpée dans un tube métallique à paroi mince sans soudure de telle manière qu'il se forme des anneaux expansibles (2, 21, 22) constitués par des bandes de matériau restantes qui s'étendent à la manière d'un réseau ou de méandres, qui sont reliés les uns aux autres par des traverses de liaison (4), où les traverses de liaison (4) qui se suivent dans la direction axiale sont disposées de manière mutuellement décalée angulairement par rapport aux précédentes ;
qui présente un premier diamètre (D₁) qui sert au transport intraluminal de la partie tubulaire, qui peut être élargi à un second diamètre variable (Dv) par le fait qu'une force radiale dirigée vers l'extérieur est appliquée depuis l'intérieur de la partie tubulaire ; et
qui est transportée dans le vaisseau sanguin et qui est élargie au moyen d'un cathéter à ballonnet,
**caractérisé en ce que**, dans chaque cas, deux anneaux (2, 21, 22) voisins sont reliés l'un à l'autre par exactement deux traverses de liaison (4) extensibles qui s'étendent de manière courbée dans la direction axiale de la partie tubulaire, qui sont disposées de manière angulairement décalée l'une par rapport à l'autre de moins de 180°.

2. Implant intraluminal selon la revendication 1, **caractérisé en ce que** les deux traverses de liaison (4) reliant entre eux dans chaque cas deux anneaux voisins sont décalées angulairement l'une par rapport à l'autre de 60 à 120°.

3. Implant intraluminal selon la revendication 1 ou 2, **caractérisé en ce que** les traverses de liaison (4) sont courbées en forme d'arcs brisés.

4. Implant intraluminal selon l'une des revendications 1 à 3, **caractérisé en ce que** chaque anneau expansible (2) consiste en au moins deux cerceaux (3) constitués par des bandes de matériau restantes qui s'étendent à la manière de méandres, qui sont reliés l'un à l'autre par une multiplicité d'autres traverses de liaison (5) extensibles qui s'étendent de manière courbée dans la direction axiale.

5. Implant intraluminal selon la revendication 4, **caractérisé en ce que** les traverses de liaison (4) entre des anneaux (2) voisins et les autres traverses de liaison (5) entre des cerceaux (3) voisins sont toutes courbées en forme d'arcs brisés et les arcs brisés entre deux anneaux (2) voisins sont tournés dans une première direction et les arcs brisés entre deux cerceaux (3) voisins sont tournés dans la direction opposée.

6. Implant intraluminal selon l'une des revendications 1 à 5, **caractérisé en ce qu'**au moins les sommets des bandes de matériau qui s'étendent en forme de méandres des anneaux (2, 21) qui sont situés le plus à l'extérieur dans la direction axiale comportent des élargissements minces (16).

7. Implant intraluminal selon la revendication 1, **caractérisé en ce que** les anneaux (21) situés le plus à l'extérieur dans la direction axiale présentent une plus grande longueur axiale que les anneaux (22) situés entre eux.

8. Implant intraluminal selon la revendication 4, **caractérisé en ce qu'**au moins les cerceaux (13) situés le plus à l'extérieur dans la direction axiale présentent une plus grande longueur axiale que les autres cerceaux (3).

9. Implant intraluminal selon la revendication 4, **caractérisé en ce que** les cerceaux (13) formant les anneaux (21) situés le plus à l'extérieur dans la direction axiale sont plus larges que les autres cerceaux (3).
